# EUROPÄISCHE PATENTSCHRIFT

(11) **EP 0 755 692 B1**
(45) Veröffentlichungstag und Bekanntmachung des Hinweises auf die Patenterteilung: **20.12.2000**
(21) Anmeldenummer: 96111279.4
(22) Anmeldetag: 12.07.1996
(51) Int. Cl.: A61M 15/02

(54) **Einrichtung zum lonisieren von Gasen**
Device for ionising gases
Appareil pour ioniser des gaz

(30) Priorität: 28.07.1995 DE 29512163 U
(43) Veröffentlichungstag der Anmeldung: 29.01.1997
(73) Patentinhaber: Medicap Medizintechnik GmbH, 35327 Ulrichstein (DE)
(72) Erfinder: Rahn, Günter, 35327 Ulrichsten - Bobenhausen (DE)
(74) Vertreter: Meier, Friedrich, Dipl.-Ing.

(56) Entgegenhaltungen:
- EP-A- 0 465 783
- DE-A- 3 612 476
- US-A- 3 717 148
- US-A- 5 396 882

## Beschreibung

Die Erfindung bezieht sich auf eine Einrichtung zum lonisieren von Gasen, insbes. von Sauerstoff für eine Sauerstofftherapie zum Anschluß an eine Atemmaske oder dergleichen unter Verwendung einer Schlauchverbindung zur Gasversorgung und eines Hochspannungserzeugers, wobei das Gas und die Hochspannung einem der Atemmaske vorgeschalteten Ionisator zugeleitet werden.

Insbesondere für eine Sauerstoff - Inhalations - Therapie wird eine definierte Mischung von medizinischem Sauerstoff und ionisiertem Sauerstoff zu präventiven und therapeutischen Zwecken eingesetzt. Der Sauerstoff wird dabei durch einen lonisator geleitet der von einem Hochspannungsgenerator über einen hochohmigen Widerstand mit einer Spannung von etwa 3000 V versorgt wird.

Da das ionisierte Gas schon über eine kurze Wegstrecke, insbesondere an Kunststoffteilen stark deionisiert wird, hat man die Teile des lonisators samt dem Hochspannungsgenerator nach DE - U - 94 13 043 unmittelbar als Baueinheit dem Anschlußstutzen einer eingesetzten Atemmaske zugeordnet. Damit wird die Ausbeute an ionisiertem Gasanteil, das der Atemmaske und damit einem Patienten zugeführt wird, wesentlich erhöht, auch wenn für den notwendigen Berührungsschutz im Bereich des Maskenanschlusses eine gelochte Abdeckung aus Kunststoff eingesetzt ist, die bekanntermaßen einen Teil der lonisierung wieder abbaut.

Alle für eine Behandlung mit ionisiertem Sauerstoff bekannt gewordenen, unmittelbar der Atemmaske zugeordneten lonisatoren sind zwar in bezug auf die Erzeugung der gewünschten lonisierung der Gase und damit auch der Versorgung der Atemmaske sehr wirksam, haben aber alle den gleichen Nachteil, nämlich den der mangelnden Hygiene und damit auch der Gefahr, Infektionskrankheiten zu übertragen. Der an eine Atemmaske angeschlossene lonisator wird durch den Träger der Atemmaske unmittelbar mit der ausgeatmeten Luft und damit auch mit Teilen der Mund - und Rachenfeuchte sowie mit Bakterien beaufschlagt, die auf den nachfolgenden Benutzer übertragen werden können.

Bei einer Ausführungsform nach EP - A - 0 465 783 ist zwischen dem Berührungsschutzgitter und dem Anschluß der Atemmaske, ein Glaskolben auf das Gehäuse der lonisators aufgesetzt. Selbst wenn dieser Glaskolben betriebsmäßig zum Zwecke der Reinigung abnehmbar wäre, könnte damit einer notwendigen Hygiene nicht Rechnung getragen werden. Nicht nur das Berührungsschutzgitter, sondern auch die lonisationskammer sind der Atemluft des Patienten (Inhalierer) unmittelbar ausgesetzt, also Aufnehmer von Bestandteilen der ausgeatmeten Luft, können also Träger von Krankheitserregern sein.

Bei einer weiteren bekannten Ausführungsform nach DE - U - 93 01 194, werden die Teile eines Sauerstoffionisators gegeneinander dauerhaft verklebt. Auf Wunsch kann jedoch auch eine Schraub - oder Klemmverbindung vorgesehen werden. Zur Berücksichtigung von Hygienemaßnahmen ist auch bei dieser bekannten Ausführungsform weder etwas vorgesehen, noch ist nach der gegebenen Lehre weder etwas machbar oder dafür brauchbar.

Der Erfindung liegt die Aufgabe zugrunde, einen für Atemmasken einsetzbaren lonisator derart zu gestalten und technisch durchzubilden, daß alle mit dem Maskenträger in direkten oder indirekten Kontakt kommenden Teile, also die der Atemmaske zugeordneten Teile, zumindest problemlos gereinigt, vorzugsweise aber desinfiziert oder sterilisiert werden können.

Gemäß der Erfindung wird die gestellte Aufgabe bei einer Einrichtung der beschriebenen Art dadurch gelöst, daß der lonisator als Hohlkörper zweiteilig derart aufgebaut ist, daß mit einem Unterteil die Anschlüsse für Gas und Hochspannung verbunden sind und in einem betriebsmäßig davon lösbaren Oberteil (lonisationskammer) eine metallische, mit einem Gasauslaß verbundene Bezugselektrode, lonisationselektroden und im Bereich des Maskenanschluß ein für das ionisierte Gas durchlässiger Berührungsschutz angeordnet sind.

Vorteilhaft ist zwischen dem Ober - und dem Unterteil eine Aufnahme für eine gasdurchlässige Filterscheibe, z. B. ein auswechselbares, Papierfilter eingesetzt. Die vorzugsweise als Bakterienfilter ausgebildete Filterscheibe ist in einem Halter angeordnet der den Gasanschluß im Unterteil mit der lonisationkammer im Oberteil verbindet. Die Filterscheibe wird durch die Bodenplatte des Oberteiles gehaltert..

Die mechanische Verbindung zwischen Ober - und Unterteil erfolgt vorteilhaft über eine drehende Verbindung, insbes. eine Rastverbindung. Der Bodenplatte des Oberteiles sind zweckmäßig zum Gasdurchlaß ringförmige Rippen zugeordnet, die vorstehende Preßflächen bilden oder ist als Lochplatte ausgebildet. Diese Bodenplatte drückt die eingelegte Filterscheibe gegen Dichtflächen im Unterteil, z.B. eingelegte, elastische O - Ringe.

An Hand der Zeichnung wird ein Ausführungsbeispiel der Erfindung beschrieben und die Wirkungsweise erläutert.

Die Fig. 1 zeigt in schematischer Darstellung einen Aufrißschnitt einer Einrichtung nach der Erfindung mit einem bajonettartigen Drehverschluß.

Die Fig. 2 zeigt eine Ausführungsform mit einer Gewindeverbindung. Gleiche Teile sind in beiden Figuren mit gleichen Bezugszeichen versehen.

Dem Unterteil 1 wird über die Schlauchverbindung 2 Gas, vorzugsweise Sauerstoff und über die elektrische Leitungen 3 und 3a Hochspannung zugeführt. Der Hochspannungserzeuger kann ebenso wie ein hochohmiger Schutzwiderstand in ein entsprechend ausgebildetes Unterteil 1 integriert sein. Dem Oberteil 4 zugewandt, ist in das Unterteil 1 eine scheibenförmige Ausnehmung 5 eingearbeitet in die als Dichtelement für die Filterscheibe 6, O - Ringe 7 eingelegt sind. Die Ausnehmung 5 ist über den Anschlußstutzen 8 mit dem Gasschlauch 2 verbunden

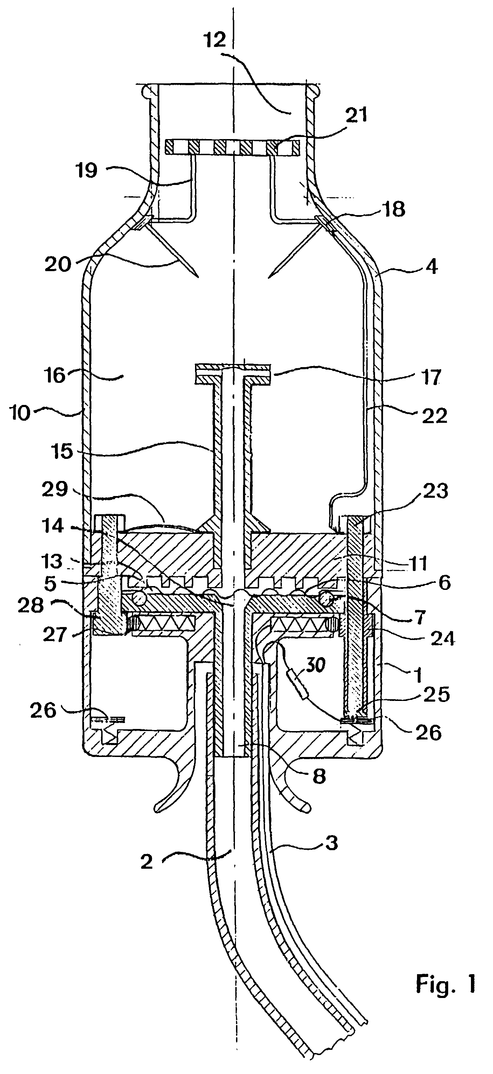

Das Oberteil 4 bildet den eigentlichen lonisator. Er besteht nach Fig. 1 aus einem Hohlkörper mit der zylinderförmigen Wandung 10, der Bodenplatte 11 und dem Anschlußstutzen 12. An die Bodenplatte 11 sind ringförmige Preßflächen 13 angeformt, durch die die Filterscheibe 6 gegen den O - Ring 7 gepreßt und ein flächiger Gasraum 14 gebildet wird. Zentrisch ist in der Bodenplatte 11 ein metallischer Rohrstutzen 15 befestigt, der den Raum 14 mit der vom Oberteil 4 gebildeten lonisationskammer 16 verbindet. Zur Reduzierung der Gasströmung ist das Kopfteil 17 des Rohrstutzens 15 mit Prallflächen oder entsprechenden Bohrungen versehen.

Im Bereich des Anschlußstutzen 12 für den Maskenanschluß, sind die Wandungen 10 eingezogen und bilden eine kegelstumpfförmige Fläche 18 an der die Träger 19 für die kranzförmig angeordneten lonisationsnadeln 20 befestigt sind. Die Träger 19 bilden auch den Halter für den unmittelbar dem Anschlußstutzen 12 zugeordneten Berührungsschutz 21. Durch die Verbindung der Träger 19 mit dem von anderen Teilen isolierten Berührungsschutz 21 können die durch den Berührungsschutz gegebenen lonisationsverluste klein gehalten werden.

Die elektrische Verbindung zwischen der elektrischen Leitung 3 (bei negativer Ionisation mit dem Minuspol verbunden) zu den lonisationsnadeln 20, erfolgt über eine an den Wandungen 10 des Oberteiles 2 anliegende Verbindung 22 die ihrerseits mit einem in der Bodenplatte 11 befestigten Kontaktstift 23 verbunden ist. Der Kontaktstift 23 ist mit einem isolierenden Kunststoffmantel 24 versehen und drückt mit seinem bodenseitigen Kontaktende 25 gegen eine federnde Kontaktlasche 26, die mit dem einen Pol der elektrischen Leitung 3 über einen hochohmigen Widerstand 30 verbunden ist.

Der andere Pol der Leitung 3 ist mit einer federbelasteten Kontaktkugel 27 verbunden, die gegen den in der Bodenplatte 11 befestigten Kontaktstift 28 drückt. Der Kontaktstift 28 ist über die Leitung 29 mit dem das Gas führenden Rohrstutzen 15 verbunden, der den Gegenpol zu den lonisationsnadeln 20 bildet.

Es ist ersichtlich, daß die elektrischen Kontaktverbindungen 23,28 unterschiedlich ausgebildet sind, so daß auch bei einem um 180° verdrehtem Aufsetzen des Oberteiles 4 auf das Unterteil 1, kein Vertauschen der durch den Anschluß der Leitung 3 gegebenen Polarität möglich ist.

Die Verbindung zwischen Unterteil 1 und Oberteil 4 kann beliebig gestaltet werden. Vorteilhaft ist es, den Kontaktstift 23 mit einer isolierenden Ummantelung 24 zu versehen und diesen Mantel zugleich als eines der Rastelemente für eine mechanische, bajonettartige Rastverbindung zwischen Oberteil 4 und Unterteil 1 zu verwenden. Das andere Rastelement wird im gezeigten Ausführungsbeispiel unmittelbar vom Kontaktstift 28 gebildet.

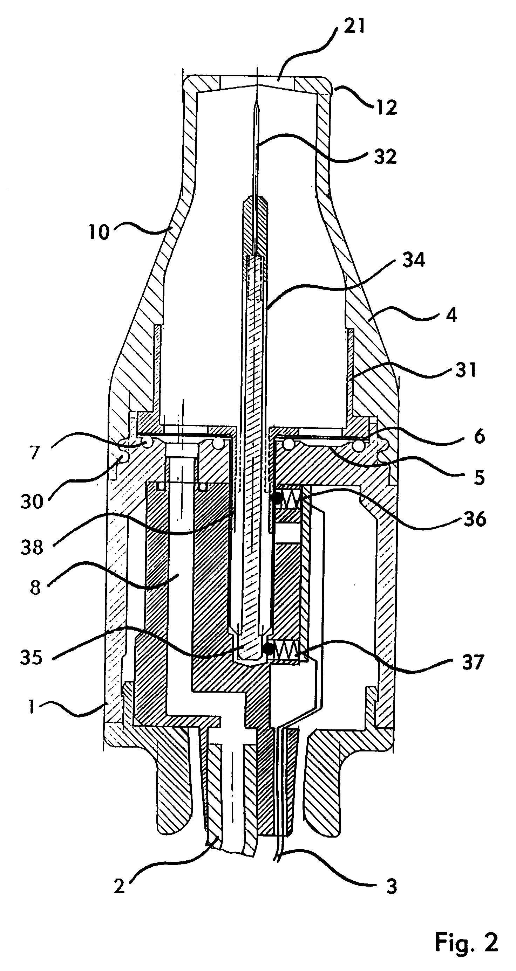

Bei der nach Fig. 2 ausgebildeten Ausführung ist das Oberteil 4 mit dem Unterteil 1 über ein Gewindestück 30 verbunden. Die zum Oberteil 4 gehörige Bodenplatte 31 ist eine metallische Lochscheibe und bildet die Bezugselektrode zu der kurz unterhalb des Berührungsschutzes 21 angeordneten lonisationselektrode 32.

Die Bodenplatte 31 ist starr mit dem Oberteil 4 verbunden. Mit der Bodenplatte 31 ist ein elektrisch leitender Rohrkörper 33 verbunden, der einen rohrförmigen Isolierkörper 34 umschließt. Der Isolierkörper 34 ist von der lonisationsnadel 32 durchsetzt, deren unteres Ende mit einer Kontaktfläche 35 versehen ist. Beim Aufsetzen des Oberteiles 4 kommen die Flächen des Rohrkörpers 33 und der lonisationsnadel 35 in Kontakt mit den federbelasteten Kontakten 36 und 37 in Eingriff.

Zumindest das Oberteil 4 und die damit verbundenen Teile, einschließlich dem gelochten Berührungsschutz 21 und den Verbindungselementen, ist aus einem temperaturfesten, mechanisch gut belastbaren, isolierenden Kunststoff, z.B. Polyetheretherketon (PEEK) oder Fluorpolymer (PTFE) gefertigt. Je nach den Anforderungen, Desinfektion oder gar Sterilität, liegt der Temperaturbereich für diese Werkstoffe ab 100 °C, bis zu Temperaturen von 280 °C.

Das abnehmbare Oberteil ermöglicht es einem Patienten oder Probanden jeweils gereinigte, desinfizierte oder sogar sterilisierte lonisatoren zur Verfügung zu stellen. Durch das betriebsmäßig mögliche Auswechseln der vorgeschalteten Filterscheibe 6 ist nicht nur sichergestellt, daß das mit den Versorgungsleitungen 2,3 verbundene Unterteil 1 mit der Atemluft des Inhalierenden nicht in Kontakt kommt, es ist sogar sichergestellt, daß auch Verunreinigungen in den zu ionisierenden Gasen sicher ausgefiltert und damit vom Inhalierenden ferngehalten werden.

## Patentansprüche

1. Einrichtung zur lonisierung von Gasen, insbesondere von Sauerstoff für eine Sauerstofftherapie, zur Verbindung mit einer Atemmaske oder dergleichen unter Verwendung einer Schlauchverbindung zur Gasversorgung und eines Hochspannungserzeugers, wobei das Gas (2) und die Hochspannung (3) einem an die Atemmaske anschließbaren lonisator zugeleitet werden, dadurch **gekennzeichnet, daß** der lonisator als Hohlkörper zweiteilig derart aufgebaut ist, daß in einem Unterteil (1) die Anschlüsse für Gas (2) und Hochspannung (3) verbunden sind und in einem betriebsmäßig davon lösbaren Oberteil (4) - Ionisationskammer - eine metallische, mit einem Gasauslaß verbundene Bezugselektrode (17,31), lonisationselektroden (20,32) und im Bereich des Maskenanschlußes (12) ein für das ionisierte Gas durchlässiger Berührungsschutz (21) angeordnet sind.

2. Einrichtung nach Anspruch 1, dadurch gekennzeichnet, daß zwischen Ober- und Unterteil (1,4) eine Aufnahme (5) für eine für Gas durchlässige Filterscheibe (6), vorzugsweise ein Bakterienfilter eingearbeitet ist, das den Gasanschluß (2) im Unterteil (1), mit der Ionisationskammer (4) verbindet.

3. Einrichtung nach Anspruch 1 und 2, dadurch gekennzeichnet, daß die mechanische Verbindung zwischen Ober- und Unterteil (1,4) über einen Drehverschluß erfolgt.

4. Einrichtung nach Anspruch 1 bis 3, dadurch gekennzeichnet, daß die elektrische Verbindung über Kontaktelemente (25,28) erfolgt, die der drehenden Verschlußbewegung über Gleitkontakte (26,27) folgen.

5. Einrichtung nach Anspruch 1 bis 4, dadurch gekennzeichnet, daß die Filterscheibe (6) durch eine der lonisationskammer (4) zugeordneten Bodenplatte (11,31) gegen die Aufnahme (5) gepreßt wird.

6. Einrichtung nach Anspruch 1 bis 5, dadurch gekennzeichnet, daß die Bodenplatte (31) als für Gas durchlässige Lochscheibe ausgebildet ist.

7. Einrichtung nach Anspruch 1 bis 6, dadurch gekennzeichnet, daß die Bodenplatte (31) als Bezugselektrode zur lonisationselektrode ausgebildet ist.

8. Einrichtung nach Anspruch 1 bis 7, dadurch gekennzeichnet, daß die elektrischen Kontaktverbindung zwischen lonisationskammer (4) und den Anschlüssen im Unterteil (1) über federbelastete Kontakte (27,36,37) erfolgt.

9. Einrichtung nach Anspruch 1 bis 8, dadurch gekennzeichnet, daß die lonisationselektroden (20,32) zusammen mit dem Berührungsschutz (21) im Bereich des Maskenanschluß (12) angeordnet ist.

10. Einrichtung nach Anspruch 1 bis 9, dadurch gekennzeichnet, daß zumindest das Oberteil (4) und der Berührungsschutz (21) aus einem hochtemperaturfesten, isolierenden Werkstoff, vorzugsweise Kunststoff wie Polyetheretherketon (PEEK) oder Fluorpolymer (PTFE) besteht, die eine Temperaturfestigkeit von mehr als 150 °C vorzugsweise mehr als 250 °C haben.

## Claims

1. Device for ionization of gases, in particular of oxygen for oxygen therapy, for connection to a breathing mask or the like using a hose connection for gas supply and a high-voltage generator, the gas (2) and the high voltage (3) being fed to an ionizer which can be attached to the breathing mask, characterized in that the ionizer is a hollow body made up of two parts and designed in such a way that the attachments for gas (2) and high voltage (3) are connected in a lower part (1) and, in an upper part (4), or ionization chamber, which can be operationally detached from the lower part (1), there is a metal reference electrode (17, 31) connected to a gas outlet, ionization electrodes (20, 32) and, in the area of the mask attachment (12), a contact protector screen (21) through which the ionized gas can pass.

2. Device according to Claim 1, characterized in that between the upper and lower parts (1, 4) there is a seat (5) for a gas-permeable filter disc (6), preferably a bacterial filter, which connects the gas attachment (2) in the lower part (1) to the ionization chamber (4).

3. Device according to Claims 1 and 2, characterized in that the mechanical connection between the upper and lower parts (1, 4) is via a rotary fastener.

4. Device according to Claims 1 to 3, characterized in that the electrical connection is via contact elements (25, 28) which follow the rotary closure movement via sliding contacts (26, 27).

5. Device according to Claims 1 to 4, characterized in that the filter disc (6) is pressed against the seat (5) by a base plate (11, 31) assigned to the ionization chamber (4).

6. Device according to Claims 1 to 5, characterized in that the base plate (31) is designed as a perforated disc which is permeable to gas.

7. Device according to Claims 1 to 6, characterized in that the base plate (31) is designed as a reference electrode for the ionization electrode.

8. Device according to Claims 1 to 7, characterized in-that the electrical contact connection between the ionization chamber (4) and the attachments in the lower part (1) is made via spring-loaded contacts (27, 36, 37).

9. Device according to Claims 1 to 8, characterized in that the ionization electrodes (20, 32) are arranged together with the contact protector screen (21) in the area of the mask attachment (12).

10. Device according to Claims 1 to 9, characterized in that at least the upper part (4) and the contact protector screen (21) are made of a high temperature-resistant, insulating material, preferably a synthetic such as polyether ether ketone (PEEK) or a fluoropolymer (PTFE), having a temperature resistance of greater than 150°C, preferably greater than 250°C.

## Revendications

1. Dispositif d'ionisation de gaz, en particulier d'oxygène pour une thérapie à l'oxygène, en vue du raccordement à un masque respiratoire ou à un appareil similaire par utilisation d'une liaison par tuyau souple en vue de l'alimentation en gaz et d'un équipement de production de haute tension, le gaz (2) et la haute tension (3) étant acheminés à un dispositif d'ionisation qui peut être raccordé au masque respiratoire, caractérisé en ce que le dispositif d'ionisation est construit sous la forme de corps creux en deux pièces de manière à ce que les raccords pour le gaz (2) et la haute tension (3) soient reliés dans une pièce inférieure (1) et à ce que soient disposées, dans une pièce supérieure (4) qui en est détachable en cours de fonctionnement - la chambre d'ionisation - une électrode de référence métallique (17, 31) reliée à une sortie de gaz, des électrodes d'ionisation (20, 32) et, dans le domaine du raccord de masque (12), une protection contre les contacts accidentels (21) perméable au gaz ionisé.

2. Dispositif selon la revendication 1, caractérisé en ce que l'on incorpore, entre la pièce supérieure et la pièce inférieure (1, 4), un adaptateur de réception (5) pour un disque à filtre perméable aux gaz (6), de préférence un filtre pour bactéries, qui relie le raccord à gaz (2) dans la pièce inférieure (1) à la chambre d'ionisation (4).

3. Dispositif selon la revendication 1 ou 2, caractérisé en ce que la liaison mécanique entre la pièce supérieure et la pièce inférieure (1, 4) se fait par l'intermédiaire d'un dispositif de verrouillage demi-tour.

4. Dispositif selon la revendication 1 à 3, caractérisé en ce que la liaison électrique se fait par l'intermédiaire d'éléments de contact (25, 28), qui suivent le mouvement de verrouillage rotatif par l'intermédiaire de contacts à frottement (26, 27).

5. Dispositif selon la revendication 1 à 4, caractérisé en ce que le disque à filtre (6) est appuyé contre l'adaptateur de réception (5) par une plaque de fond (11, 31) associée à la chambre d'ionisation (4).

6. Dispositif selon la revendication 1 à 5, caractérisé en ce que la plaque de fond (31) est formée en tant que disque ajouré perméable aux gaz.

7. Dispositif selon la revendication 1 à 6, caractérisé en ce que la plaque de fond (31) est formée en tant qu'électrode de référence par rapport à l'électrode d'ionisation.

8. Dispositif selon la revendication 1 à 7, caractérisé en ce que la liaison de contact électrique entre la chambre d'ionisation (4) et les raccords dans la pièce inférieure (1) se fait par l'intermédiaire de contacts soumis à l'action d'un ressort (27, 36, 37).

9. Dispositif selon la revendication 1 à 8, caractérisé en ce que les électrodes d'ionisation (20, 32) sont disposées dans le domaine du raccord de masque (12) conjointement à la protection contre les contacts accidentels (21).

10. Dispositif selon la revendication 1 à 9, caractérisé en ce qu'au moins la pièce supérieure (4) et la protection contre les contacts accidentels (21) se composent d'un matériau isolant, résistant aux températures élevées, de préférence d'une matière plastique comme une polyétheréthercétone (PEEK) ou un fluoropolymère (PTFE), qui possèdent une résistance à la température de plus de 150°C, de préférence de plus de 250°C.
